# EUROPEAN PATENT SPECIFICATION

(11) **EP 2 857 046 B1**
(45) Date of publication and mention of the grant of the patent: **12.06.2019**
(21) Application number: 14182100.9
(22) Date of filing: 25.08.2014
(51) Int. Cl.: B65B 55/10, A61L 2/18, A61L 2/22, B67C 7/00

(54) **STERLIZING DEVICE AND METHOD**
STERILISIERUNGSVORRICHTUNG UND STERILISIERUNGSMETHODE
DISPOSITIF ET MÉTHODE DE STÉRILISATION

(30) Priority: 07.10.2013 JP 2013209886
(43) Date of publication of application: 08.04.2015
(73) Proprietor: Mitsubishi Heavy Industries Machinery Systems, Ltd., Hyogo-ku, Kobe-shi Hyogo 652-8585 (JP)
(72) Inventor: Takesako, Yoshiyuki, Tokyo, 108-8215 (JP); Tanaka, Daisuke, Tokyo, 108-8215 (JP); Takeuchi, Yasue, Tokyo, 108-8215 (JP); Nishimura, Wataru, Tokyo, 108-8215 (JP); Ueda, Atsushi, Aichi, 453-0862 (JP); Yui, Sugihiko, Aichi, 453-0862 (JP)
(74) Representative: Hoffmann Eitle

(56) References cited:
- EP-A1- 0 303 135
- EP-A1- 1 944 263
- EP-A1- 2 008 667
- WO-A1-2013/061956
- US-A1- 2004 208 781
- US-A1- 2009 129 975
- US-A1- 2009 196 790

## Description

### BACKGROUND OF THE INVENTION

### Field of the Invention

The present invention relates to a sterilizing device which sterilizes the inside of a beverage container, typically, a plastic bottle.

### Description of the Related Art

When a container is aseptically filled with a beverage such as tea, fruit juice and coffee, it is necessary to sterilize the inside and the outside of the container before filling the container with the beverage. Therefore, a paper container or a plastic container, such as a plastic bottle, is sterilized in an aseptic filling system.

For example, a sterilizing method described in Japanese Patent No. 4526820 is known as a conventional sterilizing method. Reference may also be made to the methods disclosed in US 2009/129975 A1, EP 0 303 135 A1, US 2009/196790 A1, EP 1 944 263 A1 and WO 2013/061956 A1. Also, EP 2 008 667 A1 discloses the preamble of claims 1 and 5.

In the sterilizing method described in Japanese Patent No. 4526820, a guide member which is provided on a sterilizing agent supply pipe (a nozzle), is arranged close to a mouth portion of a beverage container (Japanese Patent No. 4526820, FIG. 5). The guide member includes a flange coaxial with the sterilizing agent supply pipe and an annular wall projecting toward the beverage container from the outer periphery of the flange.

In Japanese Patent No. 4526820, it is disclosed that a boundary portion between an outer surface and an inner surface of the mouth portion of the beverage container can be effectively and reliably sterilized by sterilizing the beverage container by use of the sterilizing agent supply pipe.

Normally, when a sterilizing agent is discharged from the nozzle inserted into the container through the mouth portion, the sterilizing agent adheres to and thereby sterilizes an inner surface of the container while moving with an airstream from a bottom portion toward the mouth portion of the container. However, since the mouth portion of the beverage container has a small opening diameter, the speed of the sterilizing agent passing through the mouth portion becomes higher than that passing through a portion below the mouth portion. Thus, the amount of the sterilizing agent adhering to the inner surface of the mouth portion is reduced. A larger amount of sterilizing agent may be discharged so as to sufficiently sterilize the inner surface of the mouth portion. In this case, however, the amount of the sterilizing agent adhering to the portion below the mouth portion becomes excessive.

The present invention has been accomplished in view of the problem as described above, and an object thereof is to provide a sterilizing device which can improve the degree of sterilization on an inner surface of a mouth portion of a beverage container.

### SUMMARY OF THE PRESENT DISCLOSURE

To achieve the above object, the present invention provides an assembly and a sterilizing method as defined in claims 1 and 5.

Since the resistor covers a mouth portion of the beverage container from above with a predetermined gap from the mouth portion, the speed of the sterilizing agent passing through the mouth portion can be reduced. Therefore, the amount of the sterilizing agent adhering to the inner peripheral surface of the mouth portion can be increased, and the degree of sterilization on the inner peripheral surface of the mouth portion can be improved.

Since the opening area A2 is made smaller than the opening area A1, the speed of the sterilizing agent passing through the first flow passage can be reduced. Therefore, the amount of the sterilizing agent adhering to the inner surface of the mouth portion can be increased.

Also, by providing the first resistor on the sterilizing agent supply pipe as recited in claims 2 and 6, the first resistor can be arranged at a predetermined position with no difficulty since the first resistor can be moved in conjunction with the movement of the sterilizing agent supply pipe.

Also, as recited in claims 3 and 7, since the second resistor is provided, and the opening area A3 of the third flow passage is made smaller than the opening area A2 of the second flow passage, the speed of the sterilizing agent passing through the first flow passage can be further reduced. Thereby, the amount of the sterilizing agent adhering to the inner surface of the mouth portion can be increased as compared to the case in which the second resistor is not provided.

Moreover, when the inside of the connection portion is formed in a rounded shape as recited in claims 4 and 8, a convection flow of the sterilizing agent discharged from the second flow passage is easily generated. When the convection flow is generated, the sterilizing agent remains in the third flow passage. The speed of the sterilizing agent discharged from the third flow passage is thereby reduced. Accordingly, the speed of the sterilizing agent passing through the first flow passage is further reduced, and the amount of the sterilizing agent adhering to the inner peripheral surface of the mouth portion can be increased.

Additionally, the present disclosure provides an assembly (not falling under the scope of the present invention as defined in the appended claims) comprising a sterilizing device and a beverage container that is an object of sterilization, wherein the sterilizing device comprises: a sterilizing agent supply pipe that is inserted into the beverage container so as to inject a sterilizing agent; and a third resistor that is provided on an outer surface of the sterilizing agent supply pipe and configured to face an inner peripheral surface of a mouth portion of the beverage container, and that is configured to suppress discharge of the sterilizing agent injected within the beverage container from the mouth portion, wherein the third resistor has a spiral shape.

The present disclosure also provides a sterilizing method (not falling under the scope of the present invention as defined in the appended claims) for a beverage container that is an object of sterilization, said beverage container having a mouth portion, said method using a sterilizing device comprising a sterilizing agent supply pipe and a third resistor provided on an outer surface of the sterilizing agent supply pipe, said third resistor having a spiral shape, said method comprising the steps of: inserting the sterilizing agent supply pipe into the beverage container in such a manner that the third resistor faces an inner peripheral surface of the mouth portion, injecting a sterilizing agent supplied by the sterilizing agent supply pipe into the beverage container, wherein the third resistor suppress discharge of the sterilizing agent injected within the beverage container from the mouth portion.

By forming a spiral flow passage by the third resistor, a distance along which the sterilizing agent passes through the mouth portion is extended, and the speed of the sterilizing agent passing through the mouth portion is reduced. Therefore, the amount of the sterilizing agent adhering to the inner peripheral surface of the mouth portion can be increased. Also, when the sterilizing agent is caused to pass through the spiral flow passage, a swirling flow is generated. A centrifugal force of the swirling flow is thereby applied to the sterilizing agent, so that the sterilizing agent more easily adheres to the inner peripheral surface of the mouth portion.

In accordance with the present invention, since the resistor is provided close to the mouth portion of the beverage container, the speed of the sterilizing agent passing through the mouth portion can be reduced. Therefore, a sufficient amount of sterilizing agent is caused to adhere to the inner peripheral surface of the mouth portion, and the degree of sterilization on the mouth portion can be improved.

### BRIEF DESCRIPTION OF THE DRAWINGS

FIG. 1 is a view illustrating the schematic configuration of an aseptic beverage filling machine according to a first embodiment of the present invention;
FIG. 2 is a view illustrating a container sterilizing device applied to the aseptic beverage filling machine in FIG. 1;
FIGS. 3A and 3B are enlarged views illustrating a region around a mouth portion of the container shown in FIG. 2;
FIGS. 4A and 4B are views for explaining the opening area of a second flow passage;
FIGS. 5A and 5B are views illustrating a second embodiment of the present invention;
FIG. 6 is a view illustrating a modification of the second embodiment of the present invention; and
FIGS. 7A and 7B are views illustrating another arrangement (not falling under the scope of the present invention as defined in the appended claims) of the present disclosure.

### DETAILED DESCRIPTION OF THE PREFERRED EMBODIMENTS OF THE PRESENT INVENTION AND OTHER ARRANGEMENTS OF THE PRESENT DISCLOSURE

### [First Embodiment]

In the following, the present invention is described based on embodiments in which a sterilizing device of the present invention is applied to an aseptic beverage filling machine.

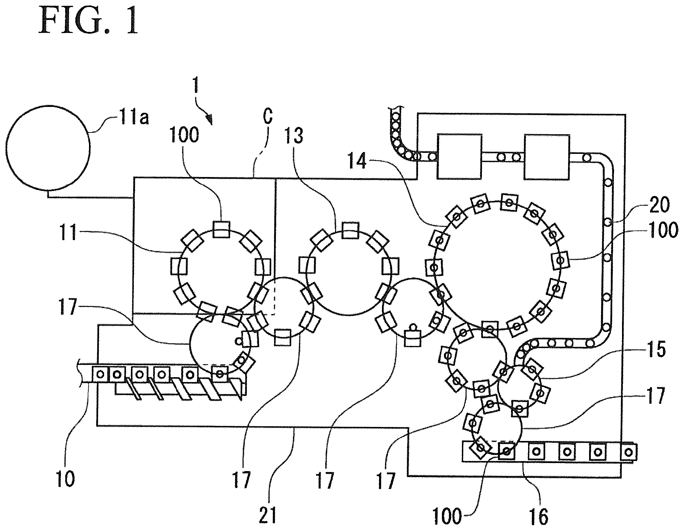

As shown in FIG. 1, an aseptic beverage filling machine 1 includes, as respective constituent elements, a feed conveyor 10 that conveys a container(s) 100 into the aseptic beverage filling machine 1, a sterilizing device 11 that sterilizes the container 100, a rinsing device 13 that rinses the container 100, a filling device 14 that fills the sterilized and rinsed container 100 with a liquid (a beverage), a capper 15 that applies a cap 20 to the container 100 filled with a beverage, and a discharge conveyor 16 that conveys the container 100 out of the aseptic beverage filling machine 1. A conveying star wheel(s) 17 is provided between the respective constituent elements. The container 100 is thereby transferred between the respective constituent elements.

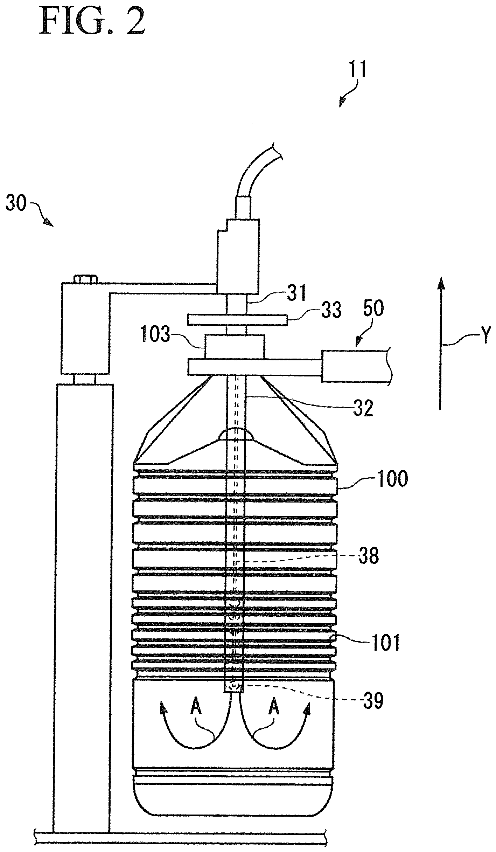

The container 100 conveyed into the aseptic beverage filling machine 1 includes a body portion 101, and a mouth portion 103 that communicates with the body portion 101 as shown in FIG. 2.

The body portion 101 has a bottomed rectangular cylindrical shape, and is filled with a beverage. The mouth portion 103 has a cylindrical shape, and a sterilizing agent supply pipe 31 is inserted into the mouth portion 103 at the time of sterilizing treatment.

A conveying route of the container 100 through the sterilizing device 11, the rinsing device 13, the filling device 14, and the capper 15 is provided on a base frame 21. The base frame 21 includes a chamber C that covers side surfaces and an upper surface of a space on the base frame 21 so as to maintain the conveying route of the container 100 through the sterilizing device 11, the rinsing device 13, the filling device 14, and the capper 15 in an aseptic environment.

A shower-type or sprinkler-type spray nozzle (not shown) is provided within the chamber C so as to spray a sterilizing agent, a rinsing liquid or the like inside the chamber C.

A drain port (not shown) for collecting the sprayed sterilizing agent, rinsing liquid or the like is also formed in the base frame 21. The collected sterilizing agent or the like is drained to an external collecting section through the drain port.

A sterilizing agent supply tank 11a is provided in association with the sterilizing device 11. The sterilizing agent supply tank 11a supplies a sterilizing agent to the chamber C and the sterilizing device 11. The sterilizing agent supplied to the chamber C is used for sterilizing the inside of the chamber C, and the sterilizing agent supplied to the sterilizing device 11 is used for sterilizing the inside and the outside of the container 100. For example, a variety of chemical agents, such as peracetic acid and hydrogen peroxide, can be applied as the sterilizing agent.

The sterilizing device 11 includes a sterilizing agent supply section 30, and a container holding section 50. The sterilizing device 11 sterilizes an inner surface of the container 100 in the course of conveying the container 100 being held by the container holding section 50.

When the sterilizing agent supply pipe 31 is inserted to a predetermined position inside the container 100 as shown in FIG. 2, the sterilizing agent supply section 30 supplies a liquid sterilizing agent to the sterilizing agent supply pipe 31. The sterilizing agent supply section 30 sterilizes the inner surface of the container 100 by injecting the sterilizing agent from the distal end of the sterilizing agent supply pipe 31 (an arrow A), and thereby causing the sterilizing agent to adhere to the inner surface of the container 100.

The sterilizing agent supply section 30 pulls up the sterilizing agent supply pipe 31 from the container 100 after injecting a predetermined amount of sterilizing agent.

The sterilizing agent supply pipe 31 includes a hollow cylindrical pipe body 32, a passage 38 that is formed within the pipe body 32 so as to allow the sterilizing agent to pass therethrough, a nozzle hole 39 that opens in the distal end of the pipe body 32 in communication with the passage 38, and a disk-shaped resistance flange (a first resistor) 33 that projects from the pipe body 32 at the proximal end.

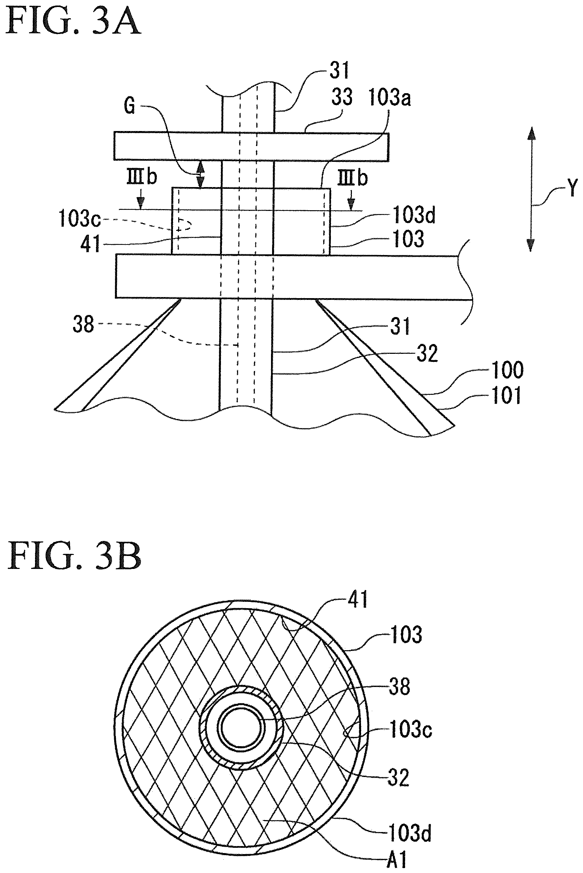

When the pipe body 32 is inserted into the container 100, a first flow passage 41 is formed between the pipe body 32 and an inner peripheral surface (an inner surface) 103c of the mouth portion 103 as shown in FIGS. 3A and 3B. The opening area of the first flow passage 41 is represented by A1. FIG. 3B is a cross-sectional view of FIG. 3A along a line IIIb-IIIb.

The sterilizing agent injected in a bottom portion of the container 100 rises and reaches the mouth portion 103 while partially adhering to the inside of the body portion 101. The sterilizing agent also partially adheres to and thereby sterilizes the inner surface 103c of the mouth portion 103. The remaining portion of the sterilizing agent is discharged to the outside of the container 100.

Since a substantial amount of the sterilizing agent is used up by adhering to the body portion 101, the amount of the sterilizing agent reaching the mouth portion 103 is small. Also, the mouth portion 103 has a small opening diameter. Thus, the speed of the sterilizing agent passing through the mouth portion 103 becomes higher than that passing through a portion below the mouth portion 103. Consequently, the amount of the sterilizing agent adhering to the mouth portion 103 inevitably becomes insufficient.

The resistance flange 33 is arranged above the mouth portion 103 on the flow passage of the sterilizing agent. The resistance flange 33 thereby serves as resistance against the flow of the sterilizing agent, and reduces the speed of discharging the sterilizing agent.

The resistance flange 33 is formed such that the diameter exceeds the opening diameter of the mouth portion 103. The resistance flange 33 is also provided perpendicular to the pipe body 32. In the sterilizing treatment, the sterilizing agent supply pipe 31 is inserted into the container 100 so as to be aligned with the axis of the mouth portion 103 (the container 100). Therefore, the resistance flange 33 is provided perpendicular to the flow passage of the sterilizing agent discharged from the first flow passage 41.

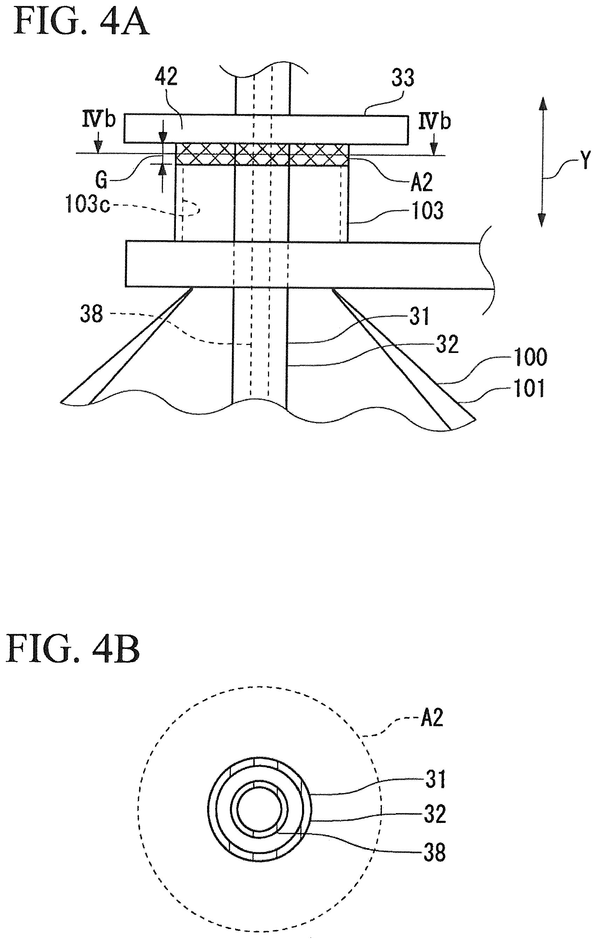

As shown in FIGS. 4A and 4B, the resistance flange 33 is arranged with a predetermined gap G in a vertical direction Y from a distal end 103a of the mouth portion 103 so as to form a second flow passage 42 between the resistance flange 33 and the distal end 103a in the sterilizing treatment. FIG. 4B is a cross-sectional view of FIG. 4A along a line IVb-IVb.

The gap G can be adjusted by an insertion position of the sterilizing agent supply pipe 31 with respect to the container 100. The sterilizing agent passing through the first flow passage 41 further passes through the second flow passage 42, and is discharged to the outside of the container 100. The opening area of the second flow passage 42 is represented by A2. In this case, the opening area A2 is calculated by multiplying a length L of the outer periphery of the mouth portion 103 by the gap G.

In the present embodiment, the gap G is set such that the opening area A2 of the second flow passage 42 is smaller than the opening area A1 of the first flow passage 41 (A1>A2).

Next, the effect of providing the resistance flange 33 on the pipe body 32 is described.

The sterilizing agent passing through the mouth portion 103 collides with the resistance flange 33. The sterilizing agent thereby loses its motion energy to be reduced in speed, and further changes its traveling direction. As described above, when the sterilizing agent collides with the resistance flange 33, the flow of the sterilizing agent is disordered within the second flow passage 42.

Also, since the opening area A2 is smaller than the opening area A1, the degree of freedom of the sterilizing agent is limited. Accordingly, the sterilizing agent becomes difficult to discharge from the second flow passage 42. That is, the speed of the sterilizing agent passing through the second flow passage 42 is reduced.

When the speed of the sterilizing agent discharged from the second flow passage 42 is reduced, the speed of the sterilizing agent discharged from the first flow passage 41 is also reduced.

As described above, when the resistance flange 33 is provided such that the opening area A2 is smaller than the opening area A1, the speed of the sterilizing agent passing through the first flow passage 41 is reduced. Therefore, the amount of the sterilizing agent adhering to the inner surface 103c is increased, and the degree of sterilization on the inner surface 103c is improved.

Accordingly, by providing the resistance flange 33 and thereby increasing the amount of the sterilizing agent adhering to the inner surface 103c, the degree of sterilization on the inner surface 103c of the mouth portion 103 can be improved.

### [Second Embodiment]

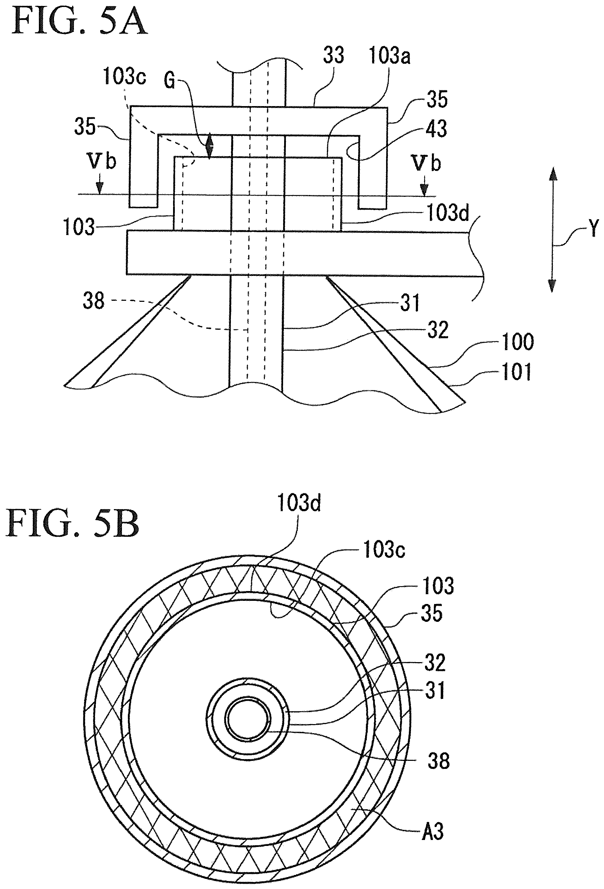

In the present embodiment, as shown in FIG. 5A, a resistance sleeve 35 (a second resistor) is formed in addition to the configuration of the first embodiment. FIG. 5B is a cross-sectional view of FIG. 5A along a line Vb-Vb.

The present embodiment has the same configuration as the first embodiment except that the resistance sleeve 35 is formed. Thus, the same members are assigned the same reference numerals, and the description thereof is omitted.

The resistance sleeve 35 is formed so as to cover the mouth portion from the periphery by hanging in the vertical direction Y from the peripheral edge of the resistance flange 33. As shown in FIG. 5B, a third flow passage 43 is formed between the resistance sleeve 35 and an outer surface 103d of the mouth portion 103 by providing the resistance sleeve 35.

Therefore, the sterilizing agent injected into the container 100 and reaching the mouth portion 103 passes through the first flow passage 41, the second flow passage 42, and the third flow passage 43, in this order and is discharged to the outside.

The opening area of the third flow passage 43 is represented by A3. In this case, the resistance sleeve 35 is provided such that the opening area A3 of the third flow passage 43 is smaller than the opening area A2 of the second flow passage 42. Therefore, the respective opening areas of the respective flow passages (41, 42, and 43) have a relationship of A1>A2>A3.

As described above, the present embodiment is featured in that the resistance sleeve 35 is provided such that the opening area A3 is smaller than the opening area A2.

The resistance sleeve 35 serves as resistance against the sterilizing agent discharged from the second flow passage 42. Furthermore, since the opening area A3 is made smaller than the opening area A2, the speed of the sterilizing agent passing through the second flow passage 42 is reduced as compared to the case in which the resistance sleeve 35 is not provided. When the speed of the sterilizing agent passing through the second flow passage 42 is reduced, the speed of the sterilizing agent passing through the first flow passage 41 is also reduced. Therefore, the amount of the sterilizing agent adhering to the inner surface 103c can be further increased. Accordingly, the degree of sterilization on the inner surface 103c can be further improved.

### [Modification]

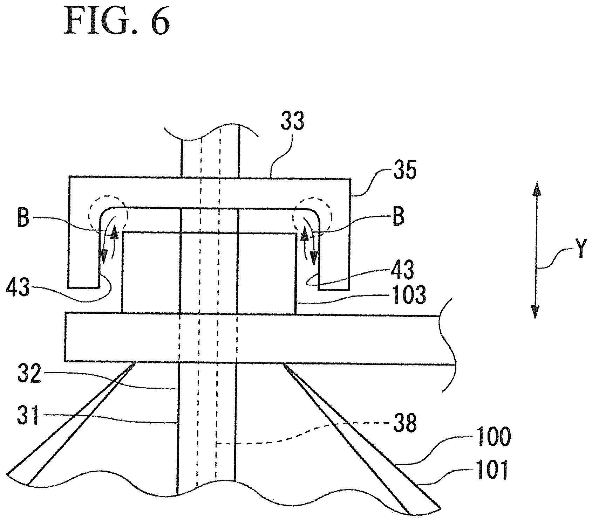

In the present embodiment, an R portion (a portion indicated by a dotted line in FIG. 6) may be formed inside a connection portion between the resistance flange 33 and the resistance sleeve 35 as shown in FIG. 6.

Since the R portion is provided in the connection portion, a convection flow of the sterilizing agent is easily generated as indicated by an arrow B when the sterilizing agent is discharged to the third flow passage 43 from the second flow passage 42. When the convection flow is generated, the amount of the sterilizing agent remaining in the third flow passage 43 is increased. Thus, the sterilizing agent becomes difficult to discharge to the outside from the third flow passage 43.

The speed of the sterilizing agent passing through the first flow passage 41 and the second flow passage 42 is thereby reduced, so that the amount of the sterilizing agent adhering to the inner surface 103c of the mouth portion 103 is increased. Accordingly, the degree of sterilization on the inner surface 103c can be further improved.

### [Other arrangement (not falling under the scope of the present invention as defined in the appended claims) of the present disclosure]

In the above embodiments, the sterilizing agent supply pipe 31 in which the resistor (the resistance flange 33) is provided on the pipe body 32 so as to be arranged outside the container 100 is described.

In the present arrangement, the resistor is provided on the pipe body 32 so as to be arranged inside the container 100.

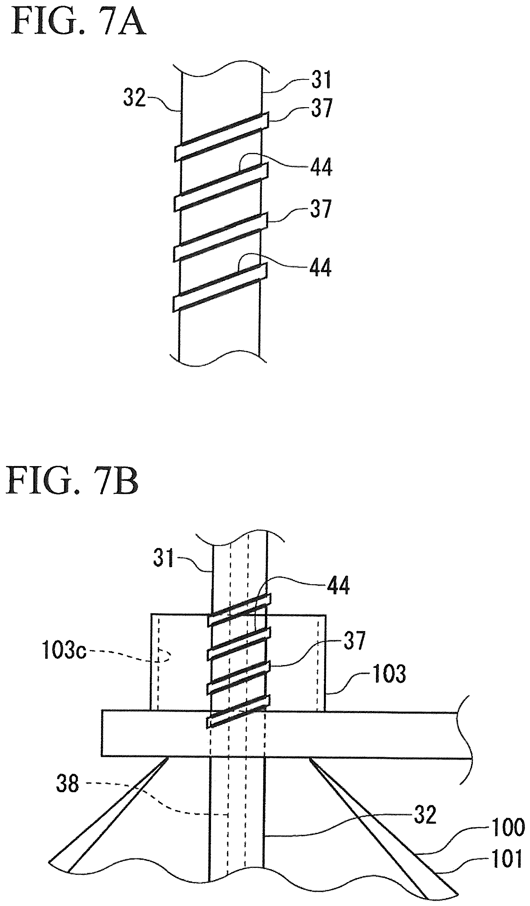

To be more specific, a spiral plate 37 is provided on an outer peripheral surface of the pipe body 32 as shown in FIGS. 7A and 7B.

The spiral plate 37 is arranged at a position of the pipe body 32 facing the inner surface 103c of the mouth portion 103 when the sterilizing agent supply pipe 31 is inserted into the container 100.

The spiral plate 37 is formed so as to be left-handed. A spiral fourth flow passage 44 is formed by the spiral plate 37 and the pipe body 32. The sterilizing agent injected into the container 100 and passing through the body portion 101 passes through the fourth flow passage 44, and is discharged to the outside.

The pitch width of the spiral plate 37 may be appropriately changed, and the spiral direction of the spiral plate 37 may be right-handed.

In accordance with the spiral plate 37 of the present arrangement, the traveling direction of the sterilizing agent linearly discharged from the body portion 101 is converted to a direction in which the spiral fourth flow passage 44 is formed. A portion of the sterilizing agent not adhering to the inner surface 103c passes through the fourth flow passage 44, and is discharged to the outside.

The spiral plate 37 serves as resistance against the sterilizing agent, and reduces the speed of the sterilizing agent passing through the mouth portion 103. Accordingly, the amount of the sterilizing agent adhering to the inner surface 103c is increased.

When the spiral plate 37 is provided, a distance along which the sterilizing agent passes through the fourth flow passage 44 becomes longer than a distance along which the sterilizing agent linearly passes through the mouth portion 103. Accordingly, it takes longer for the sterilizing agent to pass through the fourth flow passage 44.

Moreover, when the sterilizing agent is caused to pass through the fourth flow passage 44, a swirling flow is generated. A centrifugal force of the swirling flow is thereby applied to the sterilizing agent, so that the sterilizing agent is more likely to come into contact with the inner surface 103c. Thus, the sterilizing agent more easily adheres to the inner surface 103c.

As described above, by forming the fourth flow passage 44, the degree of sterilization on the inner surface 103c can be improved as compared to the case in which the sterilizing agent linearly passes through the mouth portion 103.

Although the embodiments of the present invention have been described above, the constitutions described in the embodiments described above may be also freely selected or changed into other constitutions without departing from the scope of the present invention.

For example, as long as the resistor suppresses the discharge of the sterilizing agent from the mouth portion 103 when the sterilizing agent supply pipe 31 is inserted into the container, the resistor may be provided at a position other than on the sterilizing agent supply pipe 31. It should be noted that the resistor is preferably provided on the sterilizing agent supply pipe 31. This is because the resistor can be arranged at a predetermined position with no difficulty since the resistor moves in conjunction with the movement of the sterilizing agent supply pipe 31.

The resistance flange 33 of the present embodiment is not limited to the circular shape. A rectangular or polygonal plate may be also used.

Moreover, in the present invention, the resistance flange 33 only needs to cover one portion of the distal end 103a of the mouth portion 103 so as to disturb the flow of the sterilizing agent discharged from the first flow passage 41.

In the modification of the second embodiment, a member projecting from the distal end of the resistance sleeve 35 toward the mouth portion 103 may be further provided so as to cause the sterilizing agent to remain in the third flow passage.

Moreover, in the above-described other arrangement of the present disclosure, it is only necessary to form the resistor against the sterilizing agent at a position facing the inner surface 103c. Therefore, the effect of the present disclosure can be provided even when a resistor other than the spiral plate 37, e.g., a projection formed toward the inner surface 103c is provided.

### Reference Signs List

- 1: aseptic beverage filling machine
- 10: feed conveyor
- 11: sterilizing device
- 11a: sterilizing agent supply tank
- 13: rinsing device
- 14: filling device
- 15: capper
- 16: discharge conveyor
- 17: conveying star wheel
- 20: cap
- 21: base frame
- 30: sterilizing agent supply section
- 31: sterilizing agent supply pipe
- 32: pipe body
- 33: resistance flange (first resistor)
- 35: resistance sleeve (second resistor)
- 37: spiral plate
- 41: first flow passage
- 42: second flow passage
- 43: third flow passage
- 44: fourth flow passage
- 50: container holding section
- 100: container
- 101: body portion
- 103: mouth portion
- 103a: distal end
- 103c: inner peripheral surface (an inner surface)
- 103d: outer surface
- C: chamber
- Y: vertical direction

## Claims

1. An assembly comprising a sterilizing device (11) and a beverage container (100) that is an object of sterilization,
wherein the sterilizing device comprises:
a sterilizing agent supply pipe (31) that is inserted into the beverage container (100) so as to inject a sterilizing agent; and
a first resistor (33) that covers a mouth portion (103) of the beverage container (100) from above with a predetermined gap from the mouth portion, wherein the first resistor (33) is provided such that an opening area A1 of a first flow passage (41) is formed between an outer peripheral surface of the sterilizing agent supply pipe (31) and an inner peripheral surface (103c) of the mouth portion (103), and an opening area A2 of a second flow passage (42) is formed between the first resistor (33) and an upper end of the mouth portion (103),
**characterized in that** the first resistor (33) is configured to suppress discharge of the sterilizing agent injected within the beverage container (100) from the mouth portion (103),
and **in that** the first resistor (33) is provided such that said opening areas A1 and A2 satisfy a relational expression of A1>A2.

2. The assembly according to claim 1,
wherein the first resistor (33) is provided on the sterilizing agent supply pipe (31).

3. The assembly according to claim 1 or 2,
wherein the sterilizing device (11) is provided with a second resistor (35) that hangs from a peripheral edge of the first resistor (33) so as to cover the mouth portion (103) with a predetermined gap from the mouth portion (103), and
an opening area A3 of a third flow passage (43) formed between an outer peripheral surface of the mouth portion (103) and an inner peripheral surface of the second resistor (35) satisfies a relational expression of A1>A2>A3.

4. The assembly according to claim 3,
wherein an inside of a connection portion between the first resistor (33) and the second resistor (35) is formed in a rounded shape.

5. A sterilizing method for a beverage container (100) that is an object of sterilization, said beverage container having a mouth portion (103), said method using a sterilizing device (11) comprising a sterilizing agent supply pipe (31) and a first resistor (33), said method comprising the steps of:
inserting the sterilizing agent supply pipe (31) into the beverage container (100) in such a manner that:
the first resistor (33) covers the mouth portion (103) of the beverage container (100) from above with a predetermined gap from the mouth portion (103),
an opening area A1 of a first flow passage (41) is formed between an outer peripheral surface of the sterilizing agent supply pipe (31) and an inner peripheral surface (103c) of the mouth portion (103), and an opening area A2 of a second flow passage (42) is formed between the first resistor (33) and an upper end of the mouth portion (103),
injecting a sterilizing agent supplied by the sterilizing agent supply pipe (31) into the beverage container (100),
**characterized in that** the sterilizing agent supply pipe (31) is inserted into the beverage container (100) in such a manner that said opening areas A1 and A2 satisfy a relational expression of A1>A2,
and **in that** the first resistor (33) suppresses discharge of the sterilizing agent injected within the beverage container (100) from the mouth portion (103).

6. The sterilizing method according to claim 5,
using a sterilizing device (11) wherein the first resistor (33) is provided on the sterilizing agent supply pipe.

7. The sterilizing method according to claim 5 or 6,
using a sterilizing device (11) provided with a second resistor (35) that hangs from a peripheral edge of the first resistor (33),
wherein, in the inserting step, the sterilizing agent supply pipe (31) is inserted into the beverage container (100) in such a manner that:
the second resistor (35) covers the mouth portion (103) with a predetermined gap from the mouth portion,
an opening area A3 of a third flow passage (43) is formed between an outer peripheral surface of the mouth portion (103) and an inner peripheral surface of the second resistor (33), said opening area A3 satisfying a relational expression of A1>A2>A3.

8. The sterilizing method according to claim 7,
using a sterilizing device (11) wherein an inside of a connection portion between the first resistor (33) and the second resistor (35) is formed in a rounded shape.

## Patentansprüche

1. Anordnung, umfassend eine Sterilisierungsvorrichtung (11) und einen Getränkebehälter (100), der ein Gegenstand der Sterilisierung ist,
wobei die Sterilisierungsvorrichtung umfasst:
ein Sterilisierungsmittel-Zufuhrrohr (31), das in den Getränkebehälter (100) eingeführt wird, um ein Sterilisierungsmittel einzuspritzen; und
einen ersten Widerstand (33), der einen Mündungsabschnitt (103) des Getränkebehälters (100) von oben mit einem vorbestimmten Abstand vom Mündungsabschnitt bedeckt, wobei der erste Widerstand (33) bereitgestellt ist, damit ein Öffnungsbereich A1 eines ersten Strömungskanals (41) zwischen einer äußeren Umfangsfläche des Sterilisierungsmittel-Zufuhrrohrs (31) und einer inneren Umfangsfläche (103c) des Mündungsabschnitts (103) bereitgestellt ist und ein Öffnungsbereich A2 eines zweiten Strömungskanals (42) zwischen dem ersten Widerstand (33) und einem oberen Ende des Mündungsabschnitts (103) ausgebildet ist,
**dadurch gekennzeichnet, dass** der erste Widerstand (33) konfiguriert ist, um die Abgabe des Sterilisierungsmittels, das in den Getränkebehälter (100) aus dem Mündungsabschnitt (103) eingespritzt wird, zu unterdrücken,
und dadurch, dass der erste Widerstand (33) bereitgestellt ist, sodass die Öffnungsbereiche A1 und A2 einen relationalen Ausdruck A1 > A2 erfüllen.

2. Anordnung nach Anspruch 1,
wobei der erste Widerstand (33) an dem Sterilisierungsmittel-Zufuhrrohr (31) bereitgestellt ist.

3. Anordnung nach Anspruch 1 oder 2,
wobei die Sterilisierungsvorrichtung (11) mit einem zweiten Widerstand (35) bereitgestellt ist, der von einer Umfangskante des ersten Widerstands (33) hängt, um den Mündungsabschnitt (103) mit einem vorbestimmten Abstand vom Mündungsabschnitt (103) zu bedecken, und
ein Öffnungsbereich A3 eines dritten Strömungskanals (43), der zwischen einer äußeren Umfangsfläche des Mündungsabschnitts (103) und einer inneren Umfangsfläche des zweiten Widerstands (35) gebildet ist, einen relationalen Ausdruck A1> A2> A3 erfüllt.

4. Anordnung nach Anspruch 3,
wobei eine Innenseite eines Verbindungsabschnitts zwischen dem ersten Widerstand (33) und dem zweiten Widerstand (35) in einer abgerundeten Form gebildet ist.

5. Sterilisierungsverfahren für einen Getränkebehälter (100), der ein Gegenstand der Sterilisierung ist, wobei der Getränkebehälter einen Mündungsabschnitt (103) aufweist, wobei das Verfahren eine Sterilisierungsvorrichtung (11) verwendet, die ein Sterilisierungsmittel-Zufuhrrohr (31) und einen ersten Widerstand (33) umfasst, wobei das Verfahren die folgenden Schritte umfasst:
Einführen des Sterilisierungsmittel-Zufuhrrohrs (31) in den Getränkebehälter (100) auf eine solche Weise, dass:
der erste Widerstand (33) den Mündungsabschnitt (103) des Getränkebehälters (100) von oben mit einem vorbestimmten Abstand von dem Mündungsabschnitt (103) bedeckt,
ein Öffnungsbereich A1 eines ersten Strömungskanals (41) zwischen einer äußeren Umfangsfläche des Sterilisierungsmittel-Zufuhrrohrs (31) und einer inneren Umfangsfläche (103c) des Mündungsabschnitts (103) gebildet wird, und ein Öffnungsbereich A2 eines zweiten Strömungskanals (42) zwischen dem ersten Widerstand (33) und einem oberen Ende des Mündungsabschnitts (103) gebildet wird,
Einspritzen eines Sterilisierungsmittels, das durch das Sterilisierungsmittel-Zufuhrrohr (31) in den Getränkebehälter (100) zugeführt wird,
**dadurch gekennzeichnet, dass** das Sterilisierungsmittel-Zufuhrrohr (31) auf eine solche Weise in den Getränkebehälter (100) eingesetzt ist, dass die Öffnungsbereiche A1 und A2 einen relationalen Ausdruck A1 > A2 erfüllen,
und dadurch, dass der erste Widerstand (33) die Abgabe des Sterilisierungsmittels, das in den Getränkebehälter (100) aus dem Mündungsabschnitt (103) eingespritzt wird, unterdrückt.

6. Sterilisierungsverfahren nach Anspruch 5,
unter Verwendung einer Sterilisierungsvorrichtung (11), wobei der erste Widerstand (33) an dem Sterilisierungsmittel-Zufuhrrohr bereitgestellt ist.

7. Sterilisierungsverfahren nach Anspruch 5 oder 6,
unter Verwendung einer Sterilisierungsvorrichtung (11), die mit einem zweiten Widerstand (35) bereitgestellt ist, der von einer Umfangskante des ersten Widerstands (33) hängt,
wobei in dem Einführschritt das Sterilisierungsmittel-Zufuhrrohr (31) in den Getränkebehälter (100) auf eine solche Weise eingeführt wird, dass:
der zweite Widerstand (35) den Mündungsabschnitt (103) mit einem vorbestimmten Abstand vom Mündungsabschnitt bedeckt,
ein Öffnungsbereich A3 eines dritten Strömungskanals (43) zwischen einer äußeren Umfangsfläche des Mündungsabschnitts (103) und einer inneren Umfangsfläche des zweiten Widerstands (33) gebildet wird, wobei der Öffnungsbereich A3 einen relationalen Ausdruck A1 > A2 > A3 erfüllt.

8. Sterilisierungsverfahren nach Anspruch 7,
unter Verwendung einer Sterilisierungsvorrichtung (11), wobei eine Innenseite eines Verbindungsabschnitts zwischen dem ersten Widerstand (33) und dem zweiten Widerstand (35) in einer abgerundeten Form gebildet ist.

## Revendications

1. Ensemble comprenant un dispositif de stérilisation (11) et un récipient de boisson (100) qui est un objet de stérilisation,
dans lequel le dispositif de stérilisation comprend :
un tuyau d'alimentation en agent de stérilisation (31) qui est inséré dans le récipient de boisson (100) de manière à injecter un agent de stérilisation ; et
une première résistance (33) qui couvre une partie goulot (103) du récipient de boisson (100) par au-dessus avec un espace prédéterminé par rapport à la partie goulot, dans lequel la première résistance (33) est disposée de telle sorte qu'une superficie d'ouverture A1 d'un premier passage d'écoulement (41) est formée entre une surface périphérique extérieure du tuyau d'alimentation en agent de stérilisation (31) et une surface périphérique intérieure (103c) de la partie goulot (103), et une superficie d'ouverture A2 d'un deuxième passage d'écoulement (42) est formée entre la première résistance (33) et une extrémité supérieure de la partie goulot (103),
**caractérisé en ce que** la première résistance (33) est configurée pour supprimer une décharge de l'agent de stérilisation injecté dans le récipient de boisson (100) depuis la partie goulot (103),
et **en ce que** la première résistance (33) est disposée de telle sorte que lesdites superficies d'ouverture A1 et A2 satisfont une expression relationnelle de A1>A2.

2. Ensemble selon la revendication 1,
dans lequel la première résistance (33) est disposée sur le tuyau d'alimentation en agent de stérilisation (31).

3. Ensemble selon la revendication 1 ou 2,
dans lequel le dispositif de stérilisation (11) est pourvu d'une deuxième résistance (35) qui est accrochée à un bord périphérique de la première résistance (33) de manière à couvrir la partie goulot (103) avec un espace prédéterminé par rapport à la partie goulot (103), et
une superficie d'ouverture A3 d'un troisième passage d'écoulement (43) formée entre une surface périphérique extérieure de la partie goulot (103) et une surface périphérique intérieure de la deuxième résistance (35) satisfait une expression relationnelle de A1>A2>A3.

4. Ensemble selon la revendication 3,
dans lequel un intérieur d'une partie de liaison entre la première résistance (33) et la deuxième résistance (35) est formé selon une forme arrondie.

5. Procédé de stérilisation pour un récipient de boisson (100) qui est un objet de stérilisation, ledit récipient de boisson ayant une partie goulot (103), ledit procédé utilisant un dispositif de stérilisation (11) comprenant un tuyau d'alimentation en agent de stérilisation (31) et une première résistance (33), ledit procédé comprenant les étapes de :
insertion du tuyau d'alimentation en agent de stérilisation (31) dans le récipient de boisson (100) d'une manière telle que :
la première résistance (33) couvre la partie goulot (103) du récipient de boisson (100) par au-dessus avec un espace prédéterminé par rapport à la partie goulot (103),
une superficie d'ouverture A1 d'un premier passage d'écoulement (41) est formée entre une surface périphérique extérieure du tuyau d'alimentation en agent de stérilisation (31) et une surface périphérique intérieure (103c) de la partie goulot (103), et une superficie d'ouverture A2 d'un deuxième passage d'écoulement (42) est formée entre la première résistance (33) et une extrémité supérieure de la partie goulot (103),
injection d'un agent de stérilisation apporté par le tuyau d'alimentation en agent de stérilisation (31) dans le récipient de boisson (100),
**caractérisé en ce que** le tuyau d'alimentation en agent de stérilisation (31) est inséré dans le récipient de boisson (100) d'une manière telle que lesdites superficies d'ouvertures A1 et A2 satisfont une expression relationnelle de A1>A2,
et **en ce que** la première résistance (33) supprime une décharge de l'agent de stérilisation injecté dans le récipient de boisson (100) depuis la partie goulot (103).

6. Procédé de stérilisation selon la revendication 5,
utilisant un dispositif de stérilisation (11) dans lequel la première résistance (33) est disposée sur le tuyau d'alimentation en agent de stérilisation.

7. Procédé de stérilisation selon la revendication 5 ou 6,
utilisant un dispositif de stérilisation (11) pourvu d'une deuxième résistance (35) qui est accrochée à un bord périphérique de la première résistance (33),
dans lequel, à l'étape d'insertion, le tuyau d'alimentation en agent de stérilisation (31) est inséré dans le récipient de boisson (100) d'une manière telle que :
la deuxième résistance (35) couvre la partie goulot (103) avec un espace prédéterminé par rapport à la partie goulot,
une superficie d'ouverture A3 d'un troisième passage d'écoulement (43) est formée entre une surface périphérique extérieure de la partie goulot (103) et une surface périphérique intérieure de la deuxième résistance (33), ladite superficie d'ouverture A3 satisfaisant une expression relationnelle de A1>A2>A3.

8. Procédé de stérilisation selon la revendication 7,
utilisant un dispositif de stérilisation (11) dans lequel un intérieur d'une partie de liaison entre la première résistance (33) et la deuxième résistance (35) est formé selon une forme arrondie.
